# EUROPÄISCHE PATENTANMELDUNG

(11) **EP 4 671 229 A1**
(43) Veröffentlichungstag der Anmeldung: **31.12.2025**
(21) Anmeldenummer: 25180621.2
(22) Anmeldetag: 04.06.2025
(51) Int. Cl.: C07C 1/12, C07C 29/151, C10L 3/08, C01B 3/02

(54) **VORRICHTUNG ZUR BEREITSTELLUNG EINES SYNTHESEGASGEMISCHS AUS ZUMINDEST KOHLENSTOFFDIOXID UND WASSERSTOFF**

(30) Priorität: 26.06.2024 DE 102024117973
(71) Anmelder: Everllence SE, 86153 Augsburg (DE)
(72) Erfinder: Behnke, Klaus, 69198 Schriesheim (DE); Stricker, Harald, 13437 Berlin (DE)

(57) **Zusammenfassung**

Vorrichtung (10) zur Bereitstellung eines Synthesegasgemischs aus zumindest Kohlenstoffdioxid und Wasserstoff, wobei der Vorrichtung (10) ein Massenstrom aus zumindest Kohlenstoffdioxid auf einem ersten Druckniveau und ein Massenstrom aus zumindest Wasserstoff auf einem zweiten Druckniveau bereitgestellt wird, mit einer Aufteilungseinrichtung (13) zur Aufteilung des Massenstroms aus zumindest Kohlenstoffdioxid in einen ersten und zweiten Teilmassenstrom, mit einer Teilmassenstromturbine (14) zur Entspannung des ersten Teilmassenstroms, mit einer ersten Mischungseinrichtung (16) zur Mischung des von der Teilmassenstromturbine (14) entspannten ersten Teilmassenstroms mit auf dem Massenstrom aus zumindest Wasserstoff, mit einer Verdichtungseinrichtung (17) zur Verdichtung des von der ersten Mischungseinrichtung (16) bereitgestellten Gemischs aus zumindest Kohlenstoffdioxid und Wasserstoff auf ein drittes Druckniveau, mit einer Bypassleitung (20), über welche der zweite Teilmassenstrom vorbei an der Teilmassenstromturbine (14), vorbei an der ersten Mischungseinrichtung (16) und vorbei an der Verdichtungseinrichtung (17) einer zweiten Mischungseinrichtung (21) zuführbar ist, um im Bereich der zweiten Mischungseinrichtung (17) den über die Bypassleitung (20) geführten zweiten Teilmassenstrom mit dem von der Verdichtungseinrichtung (17) verdichteten Gemisch zu mischen.

## Beschreibung

Die Erfindung betrifft eine Vorrichtung zur Bereitstellung eines Synthesegasgemischs aus zumindest Kohlenstoffdioxid und Wasserstoff insbesondere für die Erzeugung von synthetischen Kraftstoffen.

Zur Produktion von synthetischen Kraftstoffen, wie zum Beispiel zur Produktion von Methan oder Methanol, wird einerseits Wasserstoff und andererseits Kohlenstoffdioxid benötigt. Die Mischung aus zumindest Wasserstoff und Kohlenstoffdioxid wird auch als Synthesegasgemisch bezeichnet, welches einer Vorrichtung, in welcher letztendlich synthetischer Kraftstoff erzeugt wird, auf einem definierten Druckniveau bereitgestellt werden muss. So arbeiten Vorrichtungen zur Erzeugung von synthetischen Kraftstoffen, wie zum Beispiel von Methan oder Methanol auf einem Druckniveau zwischen 30 bar und 70 bar. Auf diesem Druckniveau muss ein Synthesegasgemisch, welches zumindest Kohlenstoffdioxid und Wasserstoff aufweist, bereitgestellt werden.

Das Kohlenstoffdioxid wird, vorzugsweise von einer Kohlenstoffdioxid-Bereitstellungsvorrichtung, auf einem ersten Druckniveau und der Wasserstoff wird, vorzugsweise von einer Wasserstoff-Bereitstellungsvorrichtung, auf einem zweiten Druckniveau bereitgestellt.

Bei der Wasserstoff-Bereitstellungsvorrichtung kann es sich zum Beispiel um einen Elektrolyseur handeln, der unter Verwendung von aus regenerativen Energiequellen gewonnenem elektrischen Strom Wasserstoff bereitstellt. Dieser von der Wasserstoff-Bereitstellungseinrichtung bereitgestellte Wasserstoff wird auf dem zweiten Druckniveau bereitgestellt.

Das Kohlenstoffdioxid wird auf dem ersten Druckniveau bereitgestellt, wobei es sich bei der Kohlenstoffdioxid-Bereitstellungsvorrichtung zum Beispiel um eine CCS (Carbon Capture and Storage)-Anlage oder auch um eine DAC (Direct Air Capture)-Anlage handeln kann.

Das erste Druckniveau, auf welchem das gasförmige Kohlenstoffdioxid bereitstellt wird, ist typischerweise höher als das zweite Druckniveau, auf welchem der Wasserstoff bereitstellt wird. So ist es möglich, dass das zweite Druckniveau, auf welchem der gasförmige Wasserstoff bereitstellt, Atmosphärendruck (1bar) entspricht, und dass das erste Druckniveau, auf welchem das gasförmige Kohlenstoffdioxid bereitstellt, 30 bar oder mehr beträgt.

Dann, wenn Wasserstoffgas ausgehend von dem zweiten Druckniveau auf ein Druckniveau verdichtet werden soll, auf welchem eine Vorrichtung zur Erzeugung von synthetischen Kraftstoffen arbeitet, steigt aufgrund der geringen Molmasse des Wasserstoffgases der vorrichtungstechnische Aufwand. Insbesondere wird eine große Anzahl an Verdichterstufen benötigt. Auch wird relativ viel Energie zur Verdichtung des Wasserstoffgases benötigt.

Es besteht daher Bedarf daran, das Synthesegasgemisch aus zumindest Kohlenstoffdioxid und Wasserstoff mit geringerem vorrichtungstechnischem Aufwand sowie geringerem Energiebedarf auf demjenigen Druckniveau bereitzustellen, welches insbesondere von einer Vorrichtung zur Erzeugung von synthetischen Kraftstoffen benötigt wird.

Hiervon ausgehend liegt der vorliegenden Erfindung die Aufgabe zugrunde, eine neuartige Vorrichtung zur Bereitstellung eines Synthesegasgemischs aus zumindest Kohlenstoffdioxid und Wasserstoff zu schaffen. Diese Aufgabe wird durch eine Vorrichtung nach Anspruch 1 gelöst.

Die erfindungsgemäße Vorrichtung weist eine Aufteilungseinrichtung zur Aufteilung des der Vorrichtung bereitgestellten Massenstroms aus zumindest Kohlenstoffdioxid in einen ersten Teilmassenstrom aus zumindest Kohlenstoffdioxid und einen zweiten Teilmassenstrom aus zumindest Kohlenstoffdioxid auf. Die erfindungsgemäße Vorrichtung weist ferner eine Teilmassenstromturbine zur Entspannung des ersten Teilmassenstroms aus zumindest Kohlenstoffdioxid auf. Die erfindungsgemäße Vorrichtung weist ferner eine erste Mischungseinrichtung zur Mischung des von der Teilmassenstromturbine entspannten ersten Teilmassenstroms aus zumindest Kohlenstoffdioxid mit auf dem der Vorrichtung bereitgestellten Massenstrom aus zumindest Wasserstoff auf. Die erfindungsgemäße Vorrichtung weist ferner eine Verdichtungseinrichtung zur Verdichtung des von der ersten Mischungseinrichtung bereitgestellten Gemischs aus zumindest Kohlenstoffdioxid und Wasserstoff auf ein drittes Druckniveau auf. Die erfindungsgemäße Vorrichtung weist ferner eine Bypassleitung auf, über welche der zweite Teilmassenstrom aus zumindest Kohlenstoffdioxid vorbei an der Teilmassenstromturbine, vorbei an der ersten Mischungseinrichtung und vorbei an der Verdichtungseinrichtung einer zweiten Mischungseinrichtung zuführbar bzw. in Richtung auf die zweite Mischungseinrichtung leitbar ist, um im Bereich der zweiten Mischungseinrichtung den über die Bypassleitung geführten zweiten Teilmassenstrom aus zumindest Kohlenstoffdioxid mit dem von der Verdichtungseinrichtung verdichteten Gemisch aus zumindest Kohlenstoffdioxid und Wasserstoff zu mischen.

Bei der erfindungsgemäßen Vorrichtung zur Bereitstellung des Synthesegasgemischs aus zumindest Kohlenstoffdioxid und Wasserstoff wird der insbesondere von einer Kohlenstoffdioxid-Bereitstellungsvorrichtung bereitgestellte Massenstrom aus zumindest Kohlenstoffdioxid in Teilmassenströme aufgeteilt. Ein erster Teilmassenstrom wird zur Entspannung über die Teilmassenstromturbine und anschließend im Bereich der ersten Mischungseinrichtung mit dem gasförmigen Wasserstoff, den insbesondere eine Wasserstoff-Bereitstellungsvorrichtung bereitstellt, gemischt, um nachfolgend dieses Gemisch in der Verdichtungseinrichtung zu verdichten.

Durch die Mischung des der Vorrichtung bereitgestellten Wasserstoffgases mit dem ersten Teilmassenstrom des der Vorrichtung bereitgestellten Kohlenstoffdioxids wird ein Gasgemisch bereitgestellt, welches über eine deutlich höhere Molmasse als Wasserstoffgas als solches verfügt, sodass diese Mischung aus Kohlenstoffdioxid und Wasserstoff mit geringerem vorrichtungstechnischem Aufwand sowie geringeren Kosten von der Verdichtungseinrichtung verdichtet werden kann. Zur Verdichtung der Mischung aus dem Wasserstoffgas und dem ersten Teilmassenstrom des Kohlenstoffdioxids werden weniger Verdichterstufen benötigt als für die Verdichtung des Wasserstoffgases als solchem. Ferner wird durch die polytrope Entspannung des ersten Teilmassenstroms stromaufwärts der Verdichtungseinrichtung ein Temperaturniveau für die zu verdichtende Mischung aus Wasserstoff und Kohlenstoffdioxid bereitgestellt, die eine Verdichtung des Gemischs bei geringem Leistungsbedarf ermöglicht.

Der zweite Teilmassenstrom des der Vorrichtung insbesondere von der Kohlenstoffdioxid-Bereitstellungsvorrichtung bereitgestellten Kohlenstoffdioxids wird über die Bypassleitung an der Teilmassenstromturbine, an der ersten Mischungsvorrichtung sowie an der Verdichtungsvorrichtung vorbei der zweiten Mischungseinrichtung zugeführt bzw. in Richtung auf die zweite Mischungseinrichtung geleitet, um den zweiten Teilmassenstrom stromabwärts der Verdichtungseinrichtung dem in der Verdichtungseinrichtung verdichteten Gemisch zu mischen. Dies ist auch von Vorteil, um den vorrichtungstechnischen Aufwand und Energiebedarf im Bereich der Verdichtungseinrichtung so gering wie möglich zu halten.

Die erfindungsgemäße Vorrichtung zur Bereitstellung eines Synthesegasgemischs aus zumindest Kohlenstoffdioxid und Wasserstoff erlaubt eine effiziente Bereitstellung eines Synthesegasgemischs aus zumindest Kohlenstoffdioxid und Wasserstoff insbesondere für die Erzeugung von synthetischen Kraftstoffen bei geringen Energiekosten und geringem vorrichtungstechnischem Aufwand.

Vorzugsweise weist die erfindungsgemäße Vorrichtung eine Gesamtmassenstromturbine zur Entspannung des der Vorrichtung bereitgestellten gesamten Massenstroms aus zumindest Kohlenstoffdioxid vom Druckninveau in der Bypassleitung abhängiges Druckniveau auf. Auch dies dient der Bereitstellung eines Synthesegasgemischs auf einem definierten Druckniveau bei minimalem vorrichtungstechnischem Aufwand und geringem Energiebedarf.

Vorzugsweise weist die erfindungsgemäße Vorrichtung eine stromaufwärts der Teilmassenstromturbine angeordnete Heizeinrichtung zur Erwärmung des ersten Teilmassenstroms auf. Auch dies dient der Bereitstellung eines Synthesegasgemischs auf einem gewünschten Druckniveau mit möglichst geringem vorrichtungstechnischem Aufwand und möglichst geringen Energiekosten.

Bevorzugte Weiterbildungen der Erfindung ergeben sich aus den Unteransprüchen und der nachfolgenden Beschreibung. Ausführungsbeispiele der Erfindung werden, ohne hierauf beschränkt zu sein, an Hand der Zeichnung näher erläutert. Dabei zeigt:
- Fig. 1:: eine erfindungsgemäße Vorrichtung zur Bereitstellung eines Synthesegasgemischs aus zumindest Kohlenstoffdioxid und Wasserstoff insbesondere für die Erzeugung von synthetischen Kraftstoffen;
- Fig. 2: eine Weiterbildung der Vorrichtung der Fig. 1.

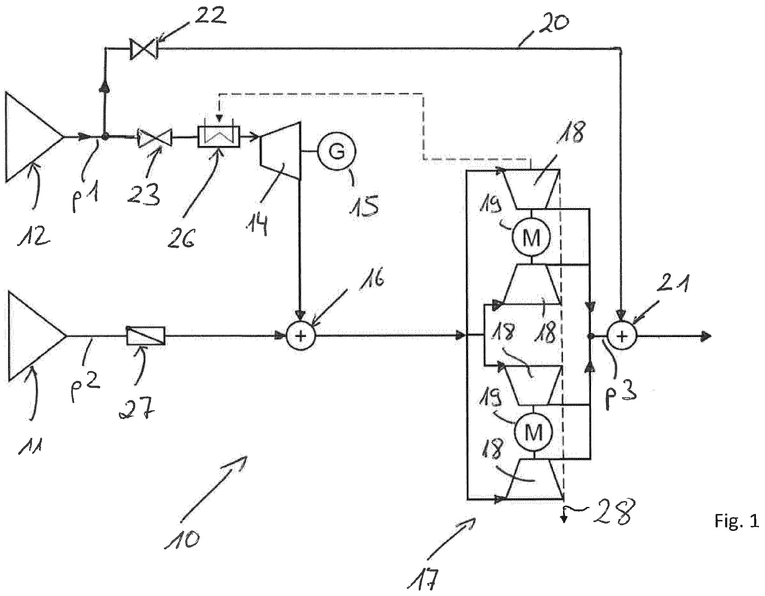

Fig. 1 zeigt stark schematisiert ein Blockschaltbild einer erfindungsgemäßen Vorrichtung 10 zur Bereitstellung eines Synthesegasgemischs aus zumindest Kohlenstoffdioxid und Wasserstoff insbesondere für die Erzeugung von synthetischen Kraftstoffen in einer nicht gezeigten Vorrichtung zur Erzeugung synthetischer Kraftstoffe. Insbesondere wird ein Synthesegasgemisch bereitgestellt, welches zur Erzeugung von Methan CH₄ oder Methanol CH₃OH verwendet werden kann.

Der erfindungsgemäßen Vorrichtung 10 wird ein Massenstrom aus zumindest Kohlenstoffdioxid auf einem ersten Druckniveau p1 und ein Massenstrom aus zumindest Wasserstoff auf einem zweiten Druckniveau p2 bereitgestellt.

In Fig. 1 stellt eine Wasserstoff-Bereitstellungseinrichtung 11 zumindest gasförmigen Wasserstoff H₂ auf dem zweiten Druckniveau p2 bereitstellt. In dem von der Wasserstoff-Bereitstellungseinrichtung 11 bereitgestellten Wasserstoff H₂ kann auch Wasserdampf H₂O und Sauerstoff O₂ enthalten sein. Bei der Wasserstoff-Bereitstellungseinrichtung 11 kann es sich um einen Elektrolyseur handeln, der unter Verwendung von aus regenerativen Energiequellen wie zum Beispiel Solarenergie oder Windenergie gewonnenem elektrischen Strom Wasserstoff erzeugt.

In Fig. 1 stellt eine Kohlenstoffdioxid-Bereitstellungseinrichtung 12 zumindest gasförmiges Kohlenstoffdioxid CO₂ auf dem ersten Druckniveau p1 bereitstellt. Bei der Kohlenstoffdioxid-Bereitstellungseinrichtung 12 kann es sich um eine CCS-Anlage oder DAC-Anlage handeln.

Die erfindungsgemäße Vorrichtung 10 zur Bereitstellung eines Synthesegasgemischs aus zumindest Kohlenstoffdioxid und Wasserstoff weist eine Aufteilungseinrichtung 13 auf, um im Ausführungsbeispiel der Fig. 1 den der Vorrichtung 10 auf dem ersten Druckniveau p1 bereitgestellten Massenstrom aus zumindest Kohlenstoffdioxid in einen ersten Teilmassenstrom und einen zweiten Teilmassenstrom aufzuteilen.

Weiterer Bestandteil der erfindungsgemäßen Vorrichtung 10 zur Bereitstellung eines Synthesegasgemischs aus zumindest Kohlenstoffdioxid und Wasserstoff ist eine Teilmassenstromturbine 14, die der polytropen Entspannung bzw. Expansion des ersten Teilmassenstroms aus zumindest Kohlenstoffdioxid dient. Der erste Teilmassenstrom wird in der Turbine 14 entspannt, wobei die Turbine 14 einen Generator 15 antreibt, welcher der Gewinnung elektrischer Energie dient.

Die erfindungsgemäße Vorrichtung 10 zur Bereitstellung eines Synthesegasgemischs aus zumindest Kohlenstoffdioxid und Wasserstoff weist ferner eine erste Mischungseinrichtung 16 auf, in welcher im Ausführungsbeispiel der Fig. 1 der in der Turbine 14 entspannte erste Teilmassenstrom aus zumindest Kohlenstoffdioxid mit dem der Vorrichtung 10 auf dem zweiten Druckniveau p2 bereitgestellten Wasserstoff gemischt wird.

Die erfindungsgemäße Vorrichtung 10 zur Bereitstellung eines Synthesegasgemischs aus zumindest Kohlenstoffdioxid und Wasserstoff weist weiterhin eine Verdichtungseinrichtung 17 auf, die der Verdichtung des durch die Mischungseinrichtung 16 bereitgestellten Gemisches auf eine drittes Druckniveau p3 dient.

Fig. 1 zeigt mehrere parallel geschaltete Verdichter 18, wobei jeweils zwei Verdichter 18 von einem gemeinsamen Motor 19 angetrieben werden. Die Anzahl der Verdichter 18 in der Verdichtungseinrichtung 17 ist rein exemplarischer Natur. Auch die Verschaltung der Verdichter 18 in der Verdichtungseinrichtung 17 ist rein exemplarischer Natur. So können auch Verdichter 18 hintereinander, also seriell, verschaltet sein.

Die erfindungsgemäße Vorrichtung 10 zur Bereitstellung eines Synthesegasgemischs aus zumindest Kohlenstoffdioxid und Wasserstoff weist weiterhin eine Bypassleitung 20 auf, über die der im Bereich der Aufteilungseinrichtung 13 abgetrennte zweite Teilmassenstrom aus Kohlenstoffdioxid vorbei an der Teilmassenstromturbine 14, vorbei an der ersten Mischeinrichtung 16 und vorbei an der Verdichtungseinrichtung 17 einer zweiten Mischungseinrichtung 21 zuführbar bzw. in Richtung auf die zweite Mischungseinrichtung 21 leitbar ist, um im Bereich der zweiten Mischungseinrichtung 21 den über die Bypassleitung 20 geführten zweiten Teilmassenstrom mit dem von der Verdichtungseinrichtung 17 verdichteten Gemisch aus dem ersten Teilmassenstrom und dem Wasserstoff zu mischen.

Wie bereits ausgeführt, dient die Aufteilungseinrichtung 13 der Aufteilung des der Vorrichtung 10 bereitgestellten Massenstrom aus zumindest gasförmigen Kohlenstoffdioxid in den über die Teilmassenstromturbine 14 zu führenden ersten Teilmassenstrom und den über die Bypassleitung 20 zu führenden zweiten Teilmassenstrom.

Die Aufteilungseinrichtung 13 weist dabei mindestens ein Ventil 22, 23 auf, wobei über die Öffnungsstellung des mindestens einen Ventils 22, 23 die Aufteilung des der Vorrichtung 10 bereitgestellten Massenstroms aus zumindest Kohlenstoffdioxid in die beiden Teilmassenströme eingestellt werden kann. In Fig. 1 sind zwei Ventile 22, 23 der Aufteilungseinrichtung 13 gezeigt. Die Aufteilungseinrichtung kann auch lediglich ein einziges Ventil 22 vorzugsweise im Bereich der Bypassleitung 20 aufweisen.

Der erste Teilmassenstrom kann dabei zwischen 25% und 75% und der zweite Teilmassenstrom zwischen 75% und 25 % des der Vorrichtung 10 bereitgestellten gesamten Massenstroms aus zumindest Kohlenstoffdioxid betragen, wobei die Summe beider Teilmassenströme 100% entspricht.

Auch ist es möglich, dass der erste Teilmassenstrom zwischen 30% und 70% und der zweite Teilmassenstrom zwischen 70% und 30% oder dass der erste Teilmassenstrom zwischen 40% und 6% und der zweite Teilmassenstrom zwischen 60% und 40% des der Vorrichtung 10 bereitgestellten gesamten Massenstroms aus zumindest Kohlenstoffdioxid entspricht.

Insbesondere ist vorgesehen, dass ein Druckniveau in der Bypassleitung 20 um 0,1 bar bis 1 bar größer ist als das dritte Druckniveau p3 des in der Verdichtungseinrichtung 17 verdichteten Gemischs unmittelbar stromabwärts der Verdichtungseinrichtung 17 oder im Bereich der zweiten Mischeinrichtung 21.

Insbesondere ist der Druck in der Bypassleitung 20 zwischen 0,1 bar und 0,6 bar oder 0,1 bar und 0,5 bar oder 0,2 bar und 0,6 bar oder 0,2 bar und 0,5 bar größer als das dritte Druckniveau p3 des in der Verdichtungseinrichtung 17 verdichteten Gemischs unmittelbar stromabwärts der Verdichtungseinrichtung 17 oder im Bereich der zweiten Mischeinrichtung 21.

In Fig. 1 ereignen sich zwischen der Aufteilungseinrichtung 13 und der Mündungsstelle der Bypassleitung 20 in die zweite Mischungseinrichtung 21 lediglich Druckverluste im Bereich des jeweiligen im Strömungsweg angeordneten Ventils sowie in der jeweiligen Strömungsleitung. Daraus folgt, dass das erste Druckniveau p1, auf welchem in Fig. 1 das Kohlenstoffdioxid bereitstellt wird, geringfügig größer ist als das Druckniveau in der Bypassleitung 20 unmittelbar stromabwärts der Verdichtungseinrichtung 17.

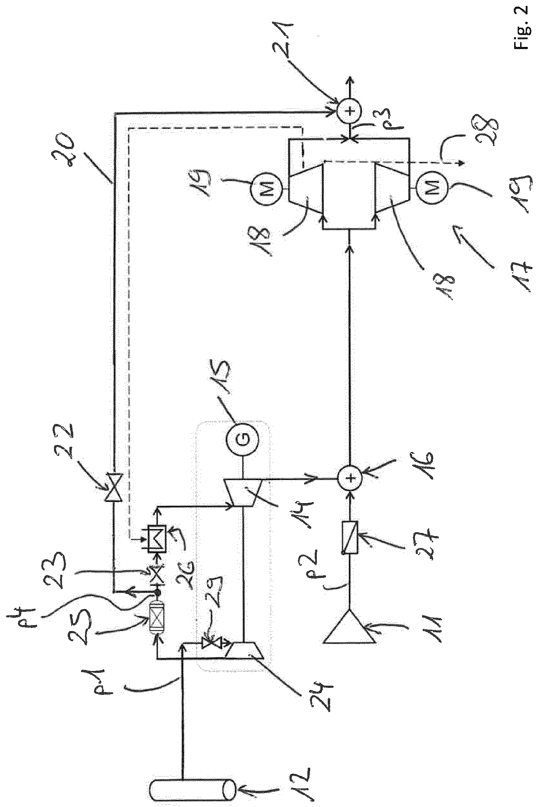

Fig. 2 zeigt eine Weiterbildung der erfindungsgemäßen Vorrichtung 10 für den Fall, in welchem das erste Druckniveau p1 deutlich oberhalb des gewünschten Druckniveaus in der Bypassleitung 20 und damit auch deutlich oberhalb des dritten Druckniveaus p3 liegt. In diesem Fall weist dann die erfindungsgemäße Vorrichtung eine Gesamtmassenstromturbine 24 auf. Über die Gesamtmassenstromturbine 24 wird der gesamte der Vorrichtung 10 bereitgestellte Massenstrom aus zumindest Kohlenstoffdioxid stromaufwärts der Aufteilungseinrichtung 13 geleitet. In Fig. 2 ist zwischen die Gesamtmassenstromturbine 24 und die Aufteilungseinrichtung 13 eine Reinigungseinrichtung 25 geschaltet, die der Reinigung des Kohlenstoffdioxids dient. Ein Ventil 29 dient der Einstellung des über die Gesamtmassenstromturbine 24 fließenden Massenstroms.

Die Aufteilungseinrichtung 13 der Fig. 2 teilt den der Vorrichtung 10 bereitgestellten Massenstrom aus zumindest Kohlenstoffdioxid ebenfalls in einen ersten Teilmassenstrom und einen zweiten Teilmassenstrom auf, jedoch stromabwärts der Gesamtmassenstromturbine 24 auf einem Druckniveau p4, das kleiner ist als das erste Druckniveau p1.

Das Druckniveau p4 im Bereich der Aufteilungseinrichtung 13 ist vom gewünschten Druckniveau in der Bypassleitung 20 abhängig, welches geringfügig oberhalb des dritten Druckniveaus p3 liegt.

Fig. 1 und 2 zeigen weiterhin eine Heizeinrichtung 26, die der Erwärmung des über die Teilmassenstromturbine 14 zu führenden ersten Teilmassenstroms unmittelbar stromaufwärts der Teilmassenstromturbine 14 und demnach stromabwärts der Aufteilungseinrichtung 13 dient. Bei dieser Heizeinrichtung 26 handelt es sich vorzugsweise um einen Wärmeaustauscher, der mit der Verdichtungseinrichtung 17 wirkverbunden ist, um im Bereich der Verdichtungseinrichtung 17 anfallende Wärme im Bereich der Heizeinrichtung 26 zu nutzen.

Bei den Ausführungsbeispielen der Fig. 1 und 2 ist zwischen die Wasserstoff-Bereitstellungseinrichtung 11 und die erste Mischungseinrichtung 16 ein Rückschlagventil 27 geschaltet. Ferner zeigen Fig. 1 und 2 eine Ableitung 28 für Kondensat.

Das Ausführungsbeispiel der Fig. 2 unterscheidet sich vom Ausführungsbeispiel der Fig. 1 auch durch die Anzahl der Verdichter 18 im Bereich der Verdichtungseinrichtung 17.

Die Erfindung erlaubt die vorteilhafte Bereitstellung eines verdichteten Synthesegasgemischs aus zumindest Kohlenstoffdioxid und Wasserstoff auf einem Druckniveau, welches insbesondere eine Vorrichtung zur Erzeugung synthetischen Kraftstoffs benötigt. Das verdichtete Synthesegasgemisch aus zumindest Kohlenstoffdioxid und Wasserstoff kann mit geringem vorrichtungstechnischem Aufwand sowie mit geringem Energiebedarf bereitgestellt werden.

Dabei ist die Aufteilung des der Vorrichtung 10 bereitgestellten Gesamtmassenstroms aus Kohlenstoffdioxid in den ersten Teilmassenstrom und den zweiten Teilmassenstrom von Bedeutung, wobei der erste Teilmassenstrom über die Teilmassenstromturbine 14 geführt und im Bereich der ersten Mischungseinrichtung 16 mit dem der Vorrichtung 10 bereitgestellten Wasserstoff gemischt wird. Der zweite Teilmassenstrom wird über die Bypassleitung 20 an der Teilmassenstromturbine 14 sowie an der Verdichtungseinrichtung 17 vorbei der zweiten Mischungseinrichtung zugeführt bzw. in Richtung auf die zweite Mischungseinrichtung 21 geführt und dort mit dem in der Verdichtungseinrichtung 14 verdichteten Gemisch aus dem ersten Teilmassenstrom und dem Wasserstoff gemischt.

### Bezugszeichenliste

- 10: Vorrichtung zur Bereitstellung eines Synthesegasgemischs
- 11: Wasserstoff-Bereitstellungseinrichtung
- 12: Kohlenstoffdioxid -Bereitstellungseinrichtung
- 13: Aufteilungseinrichtung
- 14: Teilmassenstromturbine
- 15: Generator
- 16: erste Mischungseinrichtung
- 17: Verdichtungseinrichtung
- 18: Verdichter
- 19: Motor
- 20: Bypassleitung
- 21: zweite Mischungseinrichtung
- 22: Ventil
- 23: Ventil
- 24: Gesamtmassenstromturbine
- 25: Reinigungseinrichtung
- 26: Heizeinrichtung
- 27: Rückschlagventil
- 28: Ableitung
- 29: Ventil

## Patentansprüche

1. Vorrichtung (10) zur Bereitstellung eines Synthesegasgemischs aus zumindest Kohlenstoffdioxid und Wasserstoff, insbesondere für die Erzeugung von synthetischen Kraftstoffen, wobei der Vorrichtung (10) ein Massenstrom aus zumindest Kohlenstoffdioxid auf einem ersten Druckniveau und ein Massenstrom aus zumindest Wasserstoff auf einem zweiten Druckniveau bereitgestellt wird,
mit einer Aufteilungseinrichtung (13) zur Aufteilung des Massenstroms aus zumindest Kohlenstoffdioxid in einen ersten Teilmassenstrom und einen zweiten Teilmassenstrom,
mit einer Teilmassenstromturbine (14) zur Entspannung des ersten Teilmassenstroms aus zumindest Kohlenstoffdioxid,
mit einer ersten Mischungseinrichtung (16) zur Mischung des von der Teilmassenstromturbine (14) entspannten ersten Teilmassenstroms aus zumindest Kohlenstoffdioxid mit dem Massenstrom aus zumindest Wasserstoff,
mit einer Verdichtungseinrichtung (17) zur Verdichtung des von der ersten Mischungseinrichtung (16) bereitgestellten Gemischs aus zumindest Kohlenstoffdioxid und Wasserstoff auf ein drittes Druckniveau,
mit einer Bypassleitung (20), über welche der zweite Teilmassenstrom aus zumindest Kohlenstoffdioxid vorbei an der Teilmassenstromturbine (14), vorbei an der ersten Mischungseinrichtung (16) und vorbei an der Verdichtungseinrichtung (17) einer zweiten Mischungseinrichtung (21) zuführbar ist, um im Bereich der zweiten Mischungseinrichtung (21) den über die Bypassleitung (20) geführten zweiten Teilmassenstrom aus zumindest Kohlenstoffdioxid mit dem von der Verdichtungseinrichtung (17) verdichteten Gemisch zu mischen.

2. Vorrichtung (10) nach Anspruch 1, **dadurch gekennzeichnet, dass** die Aufteilungseinrichtung (13) eingerichtet ist, den Massenstrom aus zumindest Kohlenstoffdioxid derart in den einen ersten Teilmassenstrom und den zweiten Teilmassenstrom aufzuteilen, dass der erste Teilmassenstrom zwischen 25% und 75% und der zweite Teilmassenstrom zwischen 75% und 25% des gesamten der Vorrichtung (10) bereitgestellten Massenstroms aus zumindest Kohlenstoffdioxid entspricht.

3. Vorrichtung (10) nach Anspruch 2, **dadurch gekennzeichnet, dass** der erste Teilmassenstrom zwischen 30% und 70% und der zweite Teilmassenstrom zwischen 70% und 30% oder der erste Teilmassenstrom zwischen 40% und 60% und der zweite Teilmassenstrom zwischen 60% und 40% des gesamten der Vorrichtung (10) bereitgestellten Massenstroms aus zumindest Kohlenstoffdioxid entspricht.

4. Vorrichtung (10) nach einem der Ansprüche 1 bis 3, **dadurch gekennzeichnet, dass** die Aufteilungseinrichtung (13) mindestens ein Ventil (22, 23) aufweist.

5. Vorrichtung nach einem der Ansprüche 1 bis 4, **dadurch gekennzeichnet, dass** ein Druckniveau in der Bypassleitung (20) 0,1 bar bis 1 bar oberhalb des dritten Druckniveaus liegt.

6. Vorrichtung (10) nach Anspruch 5, **dadurch gekennzeichnet, dass** das Druckniveau in der Bypassleitung (20) 0,1 bar bis 0,7 bar oder 0,1 bis 0,6 bar oder 0,2 bar bis 0,6 bar oder 0,2 bis 0,5 bar oberhalb des dritten Druckniveaus liegt.

7. Vorrichtung (10) nach Anspruche 5 oder 6, **gekennzeichnet durch** eine Gesamtmassenstromturbine (24) zur Entspannung des gesamten der Vorrichtung (10) auf dem ersten Druckniveau bereitgestellten gesamten Massenstroms aus zumindest Kohlenstoffdioxid auf ein vom Druckniveau in der Bypassleitung (20) abhängiges Druckniveau.

8. Vorrichtung (10) nach einem der Ansprüche 1 bis 7, **gekennzeichnet durch** eine stromaufwärts der Teilmassenstromturbine (14) angeordnete Heizeinrichtung (26) zur Erwärmung des ersten Teilmassenstroms.

9. Vorrichtung (10) nach Anspruch 8, **dadurch gekennzeichnet, dass** die Heizeinrichtung (26) ein Wärmeaustauscher ist, der Abwärme der Verdichtungseinrichtung (17) zur Erwärmung des ersten Teilmassenstroms nutzt.
